# EUROPÄISCHE PATENTSCHRIFT

(11) **EP 1 418 980 B1**
(45) Veröffentlichungstag und Bekanntmachung des Hinweises auf die Patenterteilung: **05.03.2008**
(21) Anmeldenummer: 02796241.4
(22) Anmeldetag: 19.08.2002
(51) Int. Cl.: A61N 1/16

(54) **VERWENDUNG EINER MISCHUNG AUS KRISTALLEN ZUR THYMUSDRÜSENSTIMULATION**
USE OF A MIXTURE OF CRYSTALS FOR STIMULATION OF THE THYMUS GLAND
UTILISATION D'UN MELANGE DE CRISTAUX POUR STIMULER LE THYMUS

(30) Priorität: 21.08.2001 DE 20113519 U; 22.11.2001 DE 20119039 U; 26.02.2002 DE 10208018
(43) Veröffentlichungstag der Anmeldung: 19.05.2004
(73) Patentinhaber: Funk, Erika, 44141 Dortmund (DE); Plebuch, Harald, 44141 Dortmund (DE)
(72) Erfinder: Funk, Erika, 44141 Dortmund (DE); Plebuch, Harald, 44141 Dortmund (DE)
(74) Vertreter: Stoffregen, Hans-Herbert
(86) Internationale Anmeldenummer: PCT/EP2002/009234
(87) Internationale Veröffentlichungsnummer: WO 2003/018115

(56) Entgegenhaltungen:
- CH-A- 687 438
- DE-A- 3 325 507
- DE-A- 4 317 884
- DE-U- 20 103 737

## Beschreibung

Die Erfindung bezieht sich auf eine Vorrichtung zur Thymusdrüsenstimulation.

Es ist bekannt, dass z. B. gelber Jaspis, Smaragd oder Serpentin eine Thymusdrüse aktiviert und anregt (Das Große Lexikon der Heilsteine, Düfte und Kräuter, Methusalem Verlagsgesellschaft mbH, Neu-Ulm, 1997, Seite 319). Auch wirkt Aquamarin, blauer Turmalin, Diamant, grüner Turmalin, Lapislazuli, Nephrit oder Peridot auf die Thymusdrüse. Messungen haben jedoch ergeben, dass nur eine eingeschränkte Wirksamkeit gegeben ist.

Das Deutsche Gebrauchsmuster 201 03 737 bezieht sich auf eine Anordnung zur Minderung des Einflusses eines elektromagnetische Felder erzeugenden Gerätes auf den Menschen. Dabei wird vorgeschlagen, eine Mischung aus Halbedelstein und Edelstein-Material oder kristallines Mineral zu verwenden, wobei Hämatit als eines der Materialien und unter anderem Bergkristall als ein anderes vorgeschlagen wird.

Die DE 43 17 884 A1 nimmt auf eine Vorrichtung zum Neutralisieren oder Abschirmen von für den menschlichen Körper schädlichen Umwelteinflüssen Bezug. Dabei wird in einen nicht metallischen Behälter ein Mineral wie Quarz eingebracht. Die DE 33 25 507 A1 hat eine Anordnung zur Entstörung pathogener Reizzonen zum Gegenstand, die Schichtungen von Graphit sowie Quarz vorsieht.

Die CH 687 438 A5 beschreibt ein Element zum Abschirmen von Erdstrahlen und von aufsteigender Feuchte in einem Mauerwerk, das einen aus elektrisch gut leitendem Material bestehenden Aufnahmekörper vorsieht, in dem körniges Mineral gefüllt ist. Hierbei kann es sich z.B. um Quarzsand handeln.

Der vorliegenden Erfindung liegt die Aufgabe zugrunde, eine Vorrichtung zur Thymusdrüsenstimulation zur Verfügung zu stellen.

Zur Lösung der Aufgabe sieht die Erfindung vor, dass in einer Aufnahme in Form eines Quaders mit in zumindest zwei Längswandungen vorhandenen Durchbrechungen Kristalle aus natürlichem Siliziumdioxid in Form von Bergkristall und natürlichem Ringsilikat in Form von Turmalin in Schüttung oder in Reihung neben- oder übereinander angeordnet sind, wobei sich das Siliziumdioxid zu dem Ringsilikat verhält wie 7:1 bis 3:1.

Bevorzugterweise sollten die Kristalle in Reibung, insbesondere übereinander angeordnet sein. Hierdurch können die Kristalle piezo-elektronische Eigenschaften entfallen, wobei der grüne Turmalin diese Eigenschaften polarisiert. Hierdurch können die zur Stimulation der Thymusdrüse erforderlichen Schwingungen erzeugt werden.

Eine besondere Wirkung ergibt sich dann, wenn das natürliche Siliziumdioxid in Form von Bergkristall und das natürliche Ringsilikat in Form von Turmalin mengenmäßig sich verhalten wie 7 : 1 bis 3 : 1.

Entsprechende Kristallmischungen zeigen überraschenderweise eine erhöhte Wirkung auf die Thymusdrüse und deren Stimulation. Auch haben nachweislich kinesiologische Messungen eine Wirkung einer entsprechenden Mischung von Kristallen auf den menschlichen Körper gezeigt. Ferner konnte festgestellt werden, dass durch eine entsprechende Mischung von na-türlichem Ringsilikat und natürlichem Siliziumdioxid, Bergkristall und insbesondere grünem Turmalin eine Einwirkung elektromagnetischer Strahlung auf den menschlichen Körper reduziert wird, insbesondere dann, wenn die Mischung sich im unmittelbaren Bereich einer Thymusdrüse befindet. Daher nimmt die Erfindung auch Bezug auf eine Vorrichtung zur Abschirmung bzw. Reduzierung elektromagnetischer Strahlung.

Hierzu werden Siliziumdioxid in Form von Bergkristall und das natürliche Ringsilikat in Form von insbesondere grünem Turmalin als Kristallmischung in einer Durchbrechungen aufweisenden Aufnahme angeordnet die ihrerseits als Schmuckstück ausgebildet und z. B. von einer Kette hängend im Bereich der Thymusdrüse angeordnet ist. Dabei sollte erwähntermaßen zwischen dem Bergkristall und dem grünem Turmalin ein Mischungsverhältnis von 7 : 1 bis 3 : 1, insbesondere 4 : 1 bestehen.

Bei dem Bergkristall bzw. grünen Turmalin sollte es sich um Kristalle insbesondere in Naturform, Chip Form, Kugelform, Nadelform oder Button-Form handeln.

Die entsprechenden Kristalle sollten dabei eine maximale Längenerstreckung L mit 2 mm ≤ 1. ≤ 3 mm aufweisen. Die Aufnahme selbst sollte aus einem Edelmetall bestehen oder dieses enthalten, wobei Silber hervorzuheben ist.

Die Geometrie der Aufnahme ist die eines Quaders, wobei insbesondere in jeder Längsseite Durchbrechungen vorgesehen sind, die eine Kreis- oder Viereck- wie Rechteck- oder Quadratgeometrie aufweisen sollten.

Typische Abmessungen eines Quaders können sein: Länge 20 - 40 mm, insbesondere etwa 30 mm bei einer Basisfläche von 3 - 7 mm x 3 - 7 mm, insbesondere 5 x 5 mm².

In den Seitenwandungen sollten jeweils zumindest drei bis sechs Durchbrechungen vorhanden sein, wobei grund- bzw. kopfflächenseitig verlaufende Durchbrechungen von einer Kette durchsetzt sein können.

Zum Befüllen eines entsprechenden Quaderkörpers wird ein umfangsseitig und bodenseitig umschlossener Körper benutzt, der nach Einbringen der Kristalle mittels eines Deckelelementes verschlossen wird, der zum Beispiel durch Kleben fixiert sein kann.

Erwähntermaßen kann die Aufnahme aus einem Edelmetall wie 925/oo Silber mit gegebenenfalls Rhodium-Anflage bestehen. Andere geeignete Materialien, die die Wirkung der Kristalle nicht versehen, sind gleichfalls denkbar.

Insbesondere sind Edelmetalle wie Silber, Gold oder Platin bzw. deren Legierungen in Betracht zu ziehen.

Ein entsprechendes Schmuckstück kann erwähntermaßen im Bereich der Thymusdrüse, also des Brustbeins angeordnet werden. Kinesiologische Armtests nach Dr. Diamond haben ergeben, dass durch entsprechende Vorrichtungen elektromagnetische Strahlung in erheblichem Umfang abgeschirmt bzw. reduziert werden kann, so dass eine Elektrosmogbelastung für eine eine entsprechende Vorrichtung tragende Personen erheblich vermindert wird

Insbesondere erfolgt jedoch eine Thymusdrüsenstimulation, sofern sich das Schmuckteil im Bereich der Thymusdrüse befindet.

Abweichend von vorbekannten Vorschlägen werden die die Abschirmwirkung bzw. Stimulation hervorrufenden Materialien - einerseits natürliches Siliziumdioxid und andererseits natürliches Ringsilikat - quasi als Schüttung oder auch ineinander bzw. übereinander angeordnet, wobei das Ringsilikat von dem Siliziumdioxid umgeben sein sollte Bevorzugterweise werden die Kristalle jedoch in Reihung angeordnet, um die piezo-elektrischen Eigenschaften voll wirksam werden zu lassen.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung von der Zeichnung zu entnehmenden bevorzugten Ausführungsbeispielen.

Es zeigen:
- Fig. 1.: eine bevorzugte Ausführungsform einer Vorrichtung zur Stimulation der Thymusdrüse und/oder zur Abschirmung bzw. Reduzierung elektromagnetischer Strahlung,
- Fig. 2: ein Gehäuse der Vorrichtung gemäß Fig. 1 und
- Fig. 3 a-c das: Gehäuse gemäß Fig. 2 mit Prinzipdarstellungen Prinzipdarstellungen in Reihung angeordneter Kristalle.

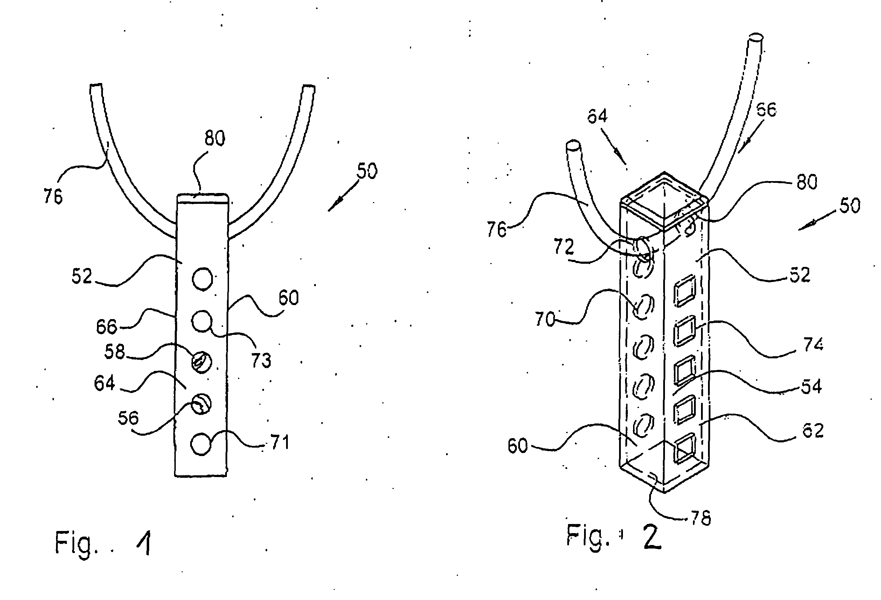

Den Fig. 1 und 2 sind bevorzugte Ausgestaltungen der erfindungsgemäßen Lehre zur Stimulation der Thymusdrüse und/oder Abschirmung bzw. Reduzierung elektromagnetischer Strahlung zu entnehmen.

Die entsprechende Vorrichtung 50 weist eine Aufnahme in Form eines Quaders 52 auf, in dessen Innenraum 54 Bergkristalle 56 und grüner Turmalin 58 eingebracht sind, wobei das Verhältnis zwischen dem Bergkristall 56 und dem grünen Turmalin 58 sich verhalten sollte zwischen 7 : 1 und 3 : 1, insbesondere 4 : 1. Dabei sind die Kristalle 56, 58 in dem Innenraum 54 des Gehäuses 52 derart angeordnet, dass der grüne Turmalin 58 zwischen Schichten des Bergkristall 56 verläuft, also der grüne Turmalin 58 sowohl unterseitig als auch oberseitig von dem Bergkristall 56 begrenzt ist.

Auch wenn die Kristalle 56, 58 in der Aufnahme als Schüllung eingebracht sein können, ist bevorzugterweise vorgesehen, dass die Kristalle 56, 58 in Reihung übereinander angeordnet sind, um die piezo-elektrischen Eigenschaften voll wirksam werden zu lassen, wobei der grüne Turmalin diese Eigenschaften polarisiert. Hierdurch können die zur Stimulation der Thymusdrüse erforderlichen Schwingungen erzeugt werden.

Sowohl der grüne Turmalin 58 als auch der Bergkristall 56 besteht aus einzelnen Kristallkörpern insbesondere in Naturform, Chip-Form, Kugelform, Nadelform oder Button-Form, wobei maximale Längenerstreckung L in etwa 3 mm ≤ L ≤ 6 mm betragen sollte.

In den Seitenwandungen 60, 62, 64, 66 sind Durchbrechungen 70, 72, 74 vorgesehen. Dabei sind bevorzugterweise in drei Seiten - im Ausführungsbeispiel in den Seiten 60, 64 und 66 - kreisförmige Durchbrechungen und in der verbleibenden Seite 62 rechteckförmige wie quadratische Durchbrechungen vorhanden.

Des Weiteren dienen die in gegenüberliegenden Wandungen - im Ausführungsbeispiel in den Wandungen 60, 66 - kopfseitig verlaufenden Durchbrechungen 72 als Ösen für eine Kette 76. Die Höhe der quaderförmigen Aufnahme 50 kann zum Beispiel 30 mm bei quadratischer Grund- bzw. Kopffläche 78, 80 betragen, die Kantenlängen von jeweils 6,5 mm aufweisen können. Diese Abmessungen sollen jedoch die erfindungsgemäße Lehre nicht einschränken.

Hinsichtlich der kreisförmigen Durchbrechungen 70, 71, 72, 73 kann ein Durchmesser von ca. 2,5 mm gewählt werden. Auch können die Kantenlängen der rechteck förmigen bzw. quadratischen Durchbrechungen 74 2,5 mm betragen. Hierdurch erfahren die Wandungen 60, 62, 64, 66 eine hinreichende Durchlässigkeit, um die Abschirm- bzw. Strahlungsreduktionswirkung der Bergkristalle 56 und grünen Turmalinkristalle 58 optimal wirken zu lassen.

Wie insbesondere auch aus der Fig. 1 ersichtlich wird, ist das Gehäuse 52 über die Wandung 80 verschließbar, die quasi die Funktion eines Deckels aufweist. Bei entfernter Kopfwandung 80 ergibt sich ein einseitig offenes Gehäuse bestehend aus den Seitenwandungen 60, 62, 64, 66 und der Bodenwandung 78, in die die Kristalle 56, 58 eingefüllt werden können. Nachdem auch die Kette durch die kopfwandseitigen Öffnungen 62 hindurchgeführt ist, kann das Gehäuse 50 über die Kopfwandung 80 verschlossen werden.

Das Gehäuse 50 besteht z. B. aus Edelmetall wie zum Beispiel 925/oo Silber, wobei zusätzlich eine Rhodium-Auflage vorgesehen sein kann.

Andere Edelmetalle wie Gold oder Platin oder Legierungen von Edelmetall kommen gleichfalls in Betracht.

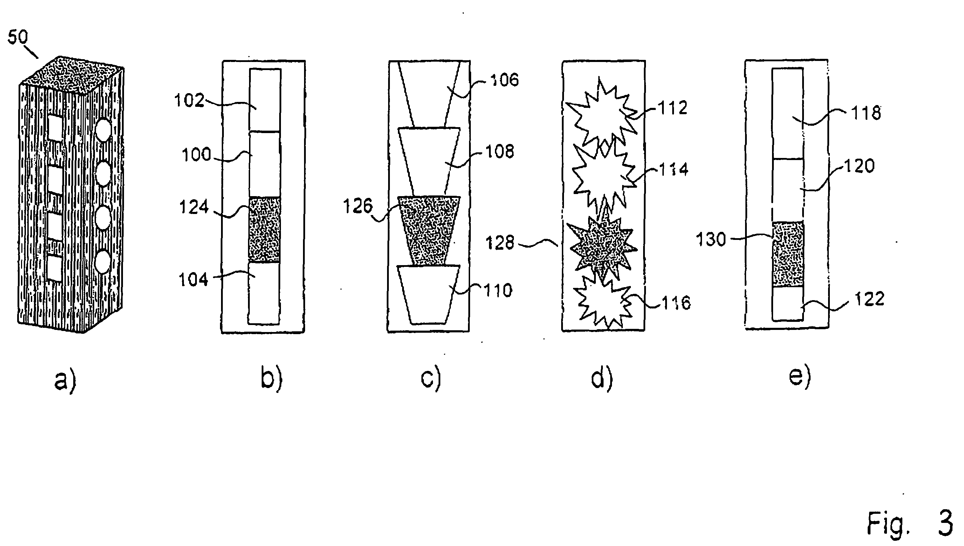

Anhand der Fig. 3a bis 3e soll noch einmal das Merkmal der in Reihung angeordneten Kristalle verdeutlicht werden. Hierdurch ist sichergestellt, dass die Kristalle auch bei Erschütterung in Kontakt zueinander bleiben, so dass sich die piezoelektrischen Eigenschaften entfalten können, wobei der grüne Turmalin diese Eigenschaften polarisiert, wodurch die gewünschten Schwingungen zur Stimulation der Thymusdrüse erzeugt werden.

In Fig. 3a ist ein ein Schmuckstück wie Anhänger darstellendes Gehäuse zur Aufnahme von Kristallen wiedergegeben, wie dieses den Fig. 1 und 2 entspricht. In den Fig. 3b bis 3e sind sodann Querschnitte des Gehäuses 50 mit in diesen in Reihung angeordneten Kristallen rein prinzipiell wiedergegeben. Dabei symbolisieren die unausgefüllten Körper 100, 102, 104, 106, 108, 110, 112, 114, 116, 118, 120, 122 Bergkristalle und die ausgefüllten Körper 124, 126, 128 und 130 grünen Turmalin.

Aus den Prinzipdarstellungen wird erkennbar, dass der Turmalin 124, 126, 128, 130 zwischen zwei Bergkristallen 100, 104 bzw. 108, 110 bzw. 120, 122 angeordnet ist. Auch erfolgt eine Berührung zwischen den Kristallen.

Die Kristalle 100, 124 in Fig. 3 können eine quadratische oder zylindrische Form aufweisen. Die Geometrie der Kristalle 106, 108, 110, 126 in Fig. 3 ist kegelstumpfförmig, wobei die kleinere Basisfläche sich auf einer größeren Basisfläche abstützt.

Die Symbole der Kristalle 112, 114, 126, 128 gemäß Fig. 3c sollen Anhäufungen von Einzelkristallen symbolisieren, die folglich jeweils eine Schültung bilden können. Unabhängig hiervon ist durch die Schüttung, die gleichfalls eine Reihung der Kristalle bildet, die erfindungsgemäße Funktion gewährleistet. Auch können die Kristalle Kugelform aufweisen.

Schließlich symbolisiert Fig. 3e, dass die Kristalle 118, 120, 122, 130 voneinander abweichende Längenerstreckungen aufweisen können.

## Patentansprüche

1. Vorrichtung zur Thymusdrüsenstimulation,
**dadurch gekennzeichnet,**
**dass** in einer Aufnahme (50) in Form eines Quaders mit in zumindest zwei Längswandungen (60, 62, 64, 66) vorhandenen Durchbrechungen (71, 72, 73, 74) Kristalle (100, 102, 104, 106, 108,110, 112, 114, 116, 118, 120, 122, 124, 126, 128, 130) aus natürlichem Siliziumdioxid in Form von Bergkristall und natürlichem Ringsilikat in Form von Turmalin in Schüttung oder in Reihung neben- oder übereinander angeordnet sind, wobei sich das Siliziumdioxid zu dem Ringsilikat verhält wie 7:1 bis 3:1.

2. Vorrichtung nach Anspruch 1,
**dadurch gekennzeichnet,**
**dass** der Turmalin grüner Turmalin (124, 126, 128, 130) ist.

3. Vorrichtung nach zumindest einem der vorhergehenden Ansprüche,
**dadurch gekennzeichnet,**
**dass** das natürliche Siliziumdioxid in Form von Bergkristall (100, 102, 104, 106, 108, 110, 112, 114, 116, 118, 120, 122) und das natürliche Ringsilikat vorzugsweise in Form von grünem Turmalin (124, 126, 128, 130) mengenmäßig sich verhalten wie 6 : 1 bis 4 : 1.

4. Vorrichtung nach zumindest einem der vorhergehenden Ansprüche,
**dadurch gekennzeichnet,**
**dass** in dem Hohlkörper die Kristalle in Form von Bergkristall (100, 102, 104, 106, 108, 110, 112, 114, 116, 118, 120, 122) und grünem Turmalin (124, 126, 128, 130) in Naturform, Chip-Form, Kugelform, Nadelform und/oder Button-Form geschichtet angeordnet sind.

5. Vorrichtung nach zumindest einem der vorhergehenden Ansprüche,
**dadurch gekennzeichnet,**
**dass** zumindest mehrere erste Körper (56) in Form des Siliziumdioxids (100, 102, 104, 106, 108, 110, 112, 114, 116, 118, 120, 122) und zumindest ein zweiter Körper in Form von natürlichem Ringsilikat (124, 126, 128, 130) in der Aufnahme (50) angeordnet sind, wobei die ersten Körper und/oder der zumindest eine zweite Körper jeweils eine maximal Längenerstreckung L mit 2 mm ≤ L ≤ 3 mm aufweisen.

6. Vorrichtung nach zumindest Anspruch 1,
**dadurch gekennzeichnet,**
**dass** die Aufnahme (50) aus Edelmetall wie Gold, Silber oder Platin oder Legierungen von Edelmetal besteht oder dieses enthält.

7. Vorrichtung nach zumindest Anspruch 6,
**dadurch gekennzeichnet,**
**dass** das Edelmetall Silber ist, das gegebenenfalls mit Rhodium beschichtet ist.

8. Vorrichtung nach zumindest Anspruch 1,
**dadurch gekennzeichnet,**
**dass** jede Längswandung (60, 62, 64, 66) der Aufnahme (50) Durchbrechungen (70, 71, 72, 73, 74) aufweist.

9. Vorrichtung nach zumindest Anspruch 1,
**dadurch gekennzeichnet,**
**dass** der Quader eine geschlossene Bodenwandung (78) und/oder Kopfwandung (80) aufweist.

10. Vorrichtung nach zumindest Anspruch 1,
**dadurch gekennzeichnet,**
**dass** die Durchbrechungen (71, 72, 73, 74) eine Kreis- und/oder Viereck- wie Quadratgeometrie aufweisen.

11. Vorrichtung nach zumindest Anspruch 1,
**dadurch gekennzeichnet,**
**dass** die Aufnahme (50) ein von einer Kette (76) wie Halskette ausgehender Anhänger ist.

## Claims

1. Device for the stimulation of the thymus gland,
**characterized in**
**that** in a receptacle (50) in form of a cuboid with openings (71, 72, 73, 74) in at least two longitudinal walls (60, 62, 64, 66) crystals (100, 102, 104, 106, 108, 110, 112, 114, 116, 118, 120, 122, 124, 126, 128, 130) of natural silicon dioxide in form of rock crystal and natural ring silicate in form of tourmaline are arranged in piles or in rows beside or on top of each other, whereby the ratio of the silicon dioxide to the ring silicate is 7:1 1 to 3:1.

2. Device according to claim 1,
**characterized in**
**that** the tourmaline is green tourmaline (124, 126, 128, 130).

3. Device according to at least one of the previous claims,
**characterized in**
**that** the natural silicon dioxide in form of a rock crystal (100, 102, 104, 106, 108, 110, 112, 114, 116, 118, 120, 122), and the natural ring silicate, preferably in form of green tourmaline (124, 126, 128, 130), have a quantitative ratio of 6:1 to 4:1.

4. Device according to at least one of the previous claims,
**characterized in**
**that** in the hollow body the crystals in form of rock crystal (100, 102, 104, 106, 108, 110, 112, 114, 116, 118, 120, 122) and green tourmaline (124, 126, 128, 130) in natural form, chip form, ball form, needle form, and/or button form are arranged in layers.

5. Device according to at least one of the previous claims,
**characterized in**
**that** at least several first bodies (56) in form of the silicon dioxide (100, 102, 104, 106, 108, 110, 112, 114, 116, 118, 120, 122) and at least a second body in form of natural ring silicate (124, 126, 128, 130) are arranged in the receptacle (50) whereby the first bodies and/or the at least one second body each have a maximum length L of 2 mm ≤ L ≤ 3 mm.

6. Device according to at least claim 1,
**characterized in**
**that** the receptacle (50) consists of or contains precious metal, such as gold, silver or platinum or alloys of precious metals.

7. Device according to at least one claim 6,
**characterized in**
**that** the precious metal is silver that, if applicable, can be coated with rhodium.

8. Device according to at least claim 1,
**characterized in**
**that** every longitudinal wall (60, 62, 64, 66) of the receptacle (50) has openings (70, 71, 72, 73, 74).

9. Device according to at least claim 1,
**characterized in**
**that** the cuboid has a closed base wall (78) and/or top wall (80).

10. Device according to at least claim 1,
**characterized in**
**that** the openings (71, 72, 73, 74) have a geometry of circles and/or quadrangles such as squares.

11. Device according to at least claim 1,
**characterized in**
**that** the receptacle (50) is a pendant hanging from a chain (76) such as a necklace.

## Revendications

1. Dispositif pour stimuler le thymus,
**caractérisé en ce que**
dans un logement (509) en forme de parallélépipède comportant des ouvertures (71, 72, 73, 74), présentes sur au moins deux parois longitudinales (60, 62, 54, 66), se trouvent, en vrac ou alignés l'un à côté de l'autre ou l'un au-dessus de l'autre, des cristaux (100, 102, 104, 106, 108, 110, 112, 114, 116, 118, 120, 122, 124, 126, 128, 130) de dioxyde de silicium naturel sous forme de cristal de roche et de cyclosilicate naturel sous forme de tourmaline, le rapport entre le dioxyde de silicium et le cyclosilicate étant de 7:1 à 3:1.

2. Dispositif selon la revendication 2,
**caractérisé en ce que**
la tourmaline est de la tourmaline verte (124, 126, 128, 130).

3. Dispositif selon au moins l'une quelconque des revendications précédentes,
**caractérisé en ce que**
le rapport quantitatif entre le dioxyde de silicium sous forme de cristal de roche (100, 102, 104, 106, 108, 110, 112, 114, 116, 118, 120, 122) et le cyclosilicate naturel, de préférence sous forme de tourmaline verte, (124, 126, 128, 130) est de 6:1 à 4:1.

4. Dispositif selon au moins l'une quelconque des revendications précédentes,
**caractérisé en ce que**
dans le corps creux, les cristaux en forme de cristal de roche (100, 102, 104, 106, 108, 110, 112, 114, 116, 118, 120, 122) et de tourmaline verte (124, 126, 128, 130) sous forme naturelle, de pastille, de bille, d'aiguille et/ou de bouton, sont disposés en couches.

5. Dispositif selon au moins l'une quelconque des revendications précédentes,
**caractérisé en ce qu'**
au moins plusieurs premiers corps (56) sous forme de dioxyde de silicium naturel (100, 102, 104, 106, 108, 110, 112, 114, 116, 118, 120, 122) et au moins un deuxième corps sous forme de cyclosilicate naturel (124, 126, 128, 130) sont disposés dans le logement (50), les premiers corps et/ou au moins un deuxième corps présentant chacun une longueur maximale L égale à 2 mm ≤ L ≤ 3 mm.

6. Dispositif selon au moins la revendication 1,
**caractérisé en ce que**
le logement (50) est en métal précieux, comme l'or, l'argent, le platine ou en alliages de métal précieux ou contient celui-ci.

7. Dispositif selon au moins la revendication 8,
**caractérisé en ce que**
le métal précieux est l'argent, le cas échéant recouvert de rhodium.

8. Dispositif selon au moins la revendication 1,
**caractérisé en ce que**
chaque paroi longitudinale (60, 62, 64, 66) du logement (50) présente des ouvertures (70, 71, 72, 73, 74).

9. Dispositif selon au moins la revendication 1,
**caractérisé en ce que**
le parallélépipède présente un dessous (78) et/ou un dessus (80) fermé.

10. Dispositif selon au moins la revendication 1,
**caractérisé en ce que**
les ouvertures (71, 72, 73, 74) présentent une géométrie circulaire et/ou à quatre côtés comme un carré.

11. Dispositif selon au moins la revendication 1,
**caractérisé en ce que**
le logement (50) est un pendentif partant d'une chaîne (76) comme une chaîne de cou.
